# EUROPEAN PATENT APPLICATION

(11) **EP 1 327 618 A1**
(43) Date of publication of application: **16.07.2003**
(21) Application number: 03004997.7
(22) Date of filing: 21.10.1999
(51) Int. Cl.: C07B 33/00, G01N 21/78, B01J 31/22, C07D 341/00, C07C 309/42, C07D 231/46

(54) **Method for analyzing hydrogen peroxide with a catalyst comprising a cyclic phenol sulfide metal complex**

(30) Priority: 22.10.1998 JP 31833398; 25.05.1999 JP 14475099
(62) Divisional of application: 99949349.7
(71) Applicant: Cosmo Research Institute, Tokyo 108-0023 (JP); COSMO OIL CO., LTD, Tokyo 105-0023 (JP)
(72) Inventor: Odo, Junichi, Okayama-shi, Okayama, 700-0086 (JP); Kawahara, Nobuko, Hiroshima-shi, Hiroshima 732-0821 (JP); Akashi, Koichi, Ookawa-gun, Kawaga 769-2702 (JP); Miyano, Sotaro, Sendai-shi, Miyagi 982-0222 (JP); Iki, Nobuhiko, 1-14, Kameoka Jutaku Daiichichiku, Sendai-shi, Miyagi 980-0865 (JP); Morohashi, Naoya, Sendai-shi, Miyagi 981-3108 (JP); Takeya, Haruhiko, deceased (JP); Miyanari, Setsuko, Cosmo Research Institute, Satte-shi, Saitama 340-0112 (JP); Kumagai, Hitoshi, Cosmo Research Institute, Satte-shi, Saitama 340-0112 (JP)
(74) Representative: HOFFMANN - EITLE

(57) **Abstract**

Provided is a novel method for analyzing a hydrogen peroxide using a metal complex having a peroxidase-like activity. A cyclic phenol sulfide metal complex is obtainable by bringing a cyclic phenol sulfide metal complex represented by the formula with at least one selected from metals of the Groups 8, 1A to 7A, 1B and 3B, and is used as a catalyst for oxidation by hydrogen peroxide. Furthermore, hydrogen peroxide is analyzed using the catalyst for hydrolysis. 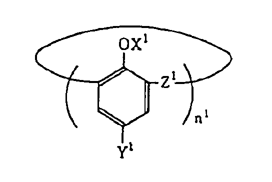 (wherein X¹ represents a hydrogen atom, a hydrocarbon group, a carboxyalkyl group, or a carbamoylalkyl group; Y¹ represents a hydrogen atom, a hydrocarbon group, a halogenated hydrocarbon group, a halogen atom, an acyl group, a hydroxyl group, a carboxyl group, an amido group, an amino group, a nitro group, a cyano group, a chlorosulfonic acid group, an alkoxysulfonyloxy group, or a sulfonic acid group; Z¹ represents Sm¹, SO, or SO₂; m¹ is an integer of from 1 to 7; n¹ is an integer of from 4 to 8).

## Description

### Technical Field

The present invention relates to a novel cyclic phenol sulfide metal complex and to a catalyst for oxidation by hydrogen peroxide and a catalyst for hydrolysis of phosphodiester which each comprises the cyclic phenol sulfide metal complex.

Also, the present invention relates to a novel method for analyzing hydrogen peroxide using the cyclic phenol sulfide metal complex.

### Background Art

Hydrogen peroxide is extensively utilized in various fields as a bleaching agent for paper/pulps and fibers, an oxidizing agent for industrial chemicals, and a bleaching agent, bactericide, or the like for medicines and foods. However, it has been pointed out that hydrogen peroxide is carcinogenic, and hydrogen peroxide generates in the living body by the action of a specific oxidase. Consequently, hydrogen peroxide is one of the substances for which quantitative analysis is necessary especially in clinical chemistry and food chemistry.

Conventionally known as techniques of quantitative analysis for hydrogen peroxide are the luminol chemiluminescence method, iodine titration method, oxygen electrode method, and the like. However, since these methods each has problems concerning influences of sample solution preparation methods on sensitivity, influences of coexistent metal ions on determination values, the time period required for analysis, and the like, a peroxidase enzymatic reaction is generally used for clinical chemical analyses, food chemical analyses, and the like.

However, the peroxidase enzymatic method has problems that the enzyme is generally expensive and that accurate determination values are difficult to obtain because the enzyme has problems concerning stability and homogeneity of quality as compared with general reagents. Investigations are being made on subjects including evaluations of compounds having an activity and usable as a substitute for the enzyme.

Namely, there is a desire for a method of quantitative analysis for hydrogen peroxide which is simple and highly suitable for determination. In other words, there is a desire for a novel compound which is utilizable in this analytical method, is excellent in chemical stability and physical stability, is inexpensive, and is utilizable as an analytical reagent having a peroxidase-like activity.

On the other hand, with the recent progress in genetic engineering, investigations are being enthusiastically made in search for a compound capable of selectively cleaving genes, such as DNA, RNA, and the like.

DNA and RNA govern the storage and manifestation of genetic information of a living being. Consequently, to enable selective cleavage of these, i.e., to develop an artificial restriction enzyme, is expected to play a highly important role in the explication of human genes and in biotechnology and cancer treatments.

In particular, if the human genome project is accomplished and the human genes are wholly explicated thereby, the selective cleavage of genes will be an indispensable important technique.

Artificial restriction enzymes are required to have a function of selectively recognizing a base sequence and a function of efficiently cleaving a nucleic acid. An artificial restriction enzyme has so far been reported which functions to cleave DNA by linking an iron complex to a recognizing site of a base sequence and breaking the sugar chain by the action of the hydroxy radical generated by the complex. However, this artificial enzyme has unsolved chemical structure of cleavage ends and is difficult to extensively utilize in this field.

DNA is a polymer consisting of deoxyribonucleoside linked through a phosphodiester bond, and the nucleic acid is desired to be cleaved by the hydrolysis of the phosphodiester bond as in the case with natural restriction enzymes.

The phosphodiester bonds in DNA are extremely stable and it has long been impossible to nonenzymatically hydrolyze these. However, Komiyama *et al.* recently reported that the bonds can be hydrolyzed with a lanthanide complex (*Kagaku, 48*(9): 654 (1993), *etc*.). As a result of these, considerable progress has been made with respect to activity in phosphodiester hydrolysis. However, it is said that there still are many unsolved problems concerning the fact that the activity is obtained under limited conditions, and concerning influences of the nucleic acid bases adjacent to the phosphodiester, hybridization with a recognized site in a base sequence, *etc*.

Consequently, there is still a desire for an artificial restriction enzyme material having high activity and selectivity in phosphodiester hydrolysis under physiological conditions.

### Disclosure of the Invention

Accordingly, an object of the present invention is to provide a novel metal complex, in particular, a metal complex which is excellent in chemical stability and physical stability, can be produced by a simple process, is inexpensive, can be used in applications, such as catalysts for various chemical reactions, information/electronic materials, and the like, and especially has a peroxidase-like activity and a high activity in phosphodiester hydrolysis.

Another object of the present invention is to provide a novel method for analyzing hydrogen peroxide using the metal complex having a peroxidase-like activity.

That is, the present invention provides a cyclic phenol sulfide metal complex represented by formula (1): 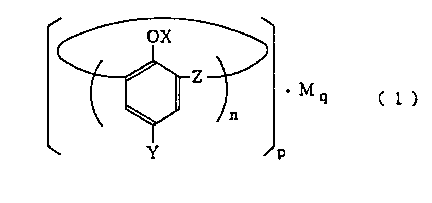 (wherein X represents a hydrogen atom, a hydrocarbon group, a carboxyalkyl group, or a carbamoylalkyl group; Y represents a hydrogen atom, a hydrocarbon group, a halogenated hydrocarbon group, a halogen atom, an acyl group, a hydroxyl group, a carboxyl group, an amido group, an amino group, a nitro group, a cyano group, a chlorosulfonic acid group, an alkoxysulfonyloxy group, or a sulfonic acid group; Z represents Sm, SO, or SO₂; m represents an integer of from 1 to 7; n represents an integer of from 4 to 8; M represents a metal of the Groups 8, 1A to 7A, 1B or 3B; and p and q represent a composition ratio and an integer of 1 or more).

Furthermore, the present invention provides a process for producing the cyclic phenol sulfide represented by formula (1), comprising bringing a cyclic alkylphenol sulfide represented by formula (2): 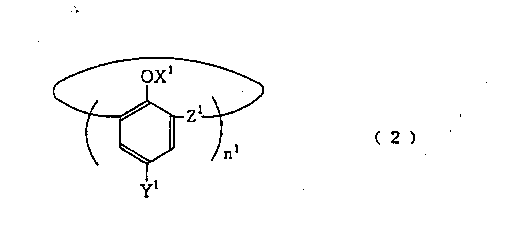 (wherein X¹ represents a hydrogen atom, a hydrocarbon group, a carboxyalkyl group, or a carbamoylalkyl group; Y¹ represents a hydrogen atom, a hydrocarbon group, a halogenated hydrocarbon group, a halogen atom, an acyl group, a hydroxyl group, a carboxyl group, an amido group, an amino group, a nitro group, a cyano group, a chlorosulfonic acid group, an alkoxysulfonyloxy group, or a sulfonic acid group; Z¹ represents Sm¹, SO, or SO₂; m¹ is an integer of from 1 to 7; n¹ is an integer of from 4 to 8) into contact with at least one selected from metals of the Group 8, 1A to 7A, 1B and 3B.

Moreover, the present invention provides a composition for a catalyst reaction, comprising the cyclic phenol sulfide metal complex described in the above formula (1) and a support.

Also, the present invention provides a composition having a catalytic activity for an oxidation by hydrogen peroxide, comprising a cyclic phenol sulfide metal complex represented by formula (3): 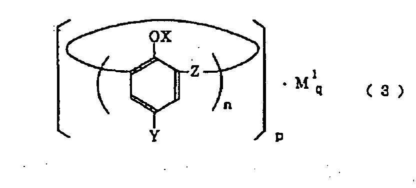 (wherein X represents a hydrogen atom, a hydrocarbon-group, a carboxyalkyl group, or a carbamoylalkyl group; Y represents a hydrogen atom, a hydrocarbon group, a halogenated hydrocarbon group, a halogen atom, an acyl group, a hydroxyl group, a carboxyl group, an amido group, an amino group, a nitro group, a cyano group, a chlorosulfonic acid group, an alkoxysulfonyloxy group, or a sulfonic acid group; Z represents Sm, SO, or SO₂; m represents an integer of from 1 to 7; n represents an integer of from 4 to 8; M¹ represents a transition metal or a rear earth metal; and p and q represent a composition ratio and an integer of 1 or more) and a support.

Still furthermore, the present invention provides a novel method for analyzing hydrogen peroxide using the cyclic phenol sulfide metal complex having a catalytic activity in oxidations by hydrogen peroxide or the cyclic phenol sulfide metal complex having a catalytic activity in oxidations by hydrogen peroxide, which has been mixed with or supported on a solid support.

Still moreover, the present invention provides a composition for catalyzing hydrolysis of phosphodiester comprising the cyclic phenol sulfide metal complex represented by formula (3) described above and a support.

### Brief Description of the Drawings

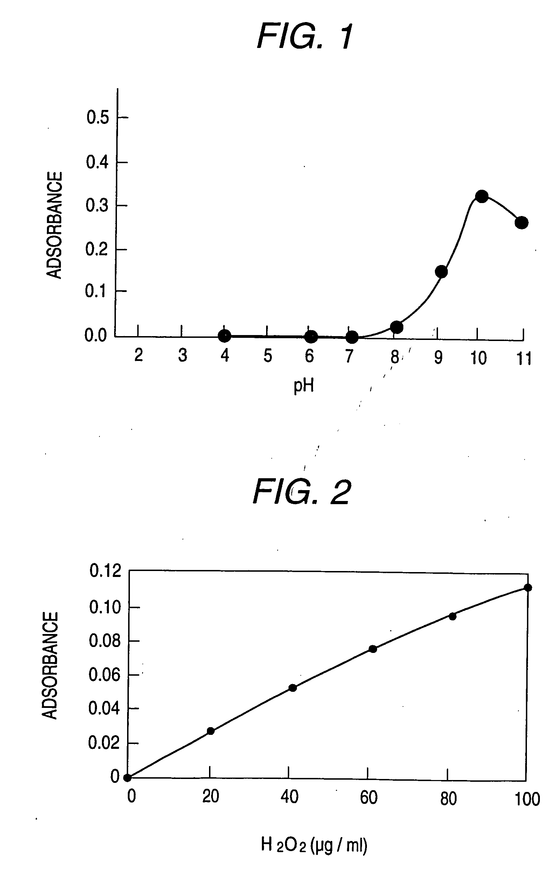

Fig. 1 is a graph showing the results obtained by determining the absorbance at 505 nm for each pH which indicates the amount of a quinoid type dye yielded by reacting hydrogen peroxide, 4-aminoantipyrine (4-AAP), and phenol using the catalytic function of a cyclic phenol sulfide sulfonic acid iron complex of the present invention.
Fig. 2 is a graph showing the results obtained by determining the absorbance at 505 nm, at hydrogen peroxide concentrations of from 0 to 100 µg/ml, which indicates the amount of a quinoid type dye yielded by reacting hydrogen peroxide, 4-AAP, and phenol using the catalytic function of a cyclic phenol sulfide sulfonic acid iron complex of the present invention.

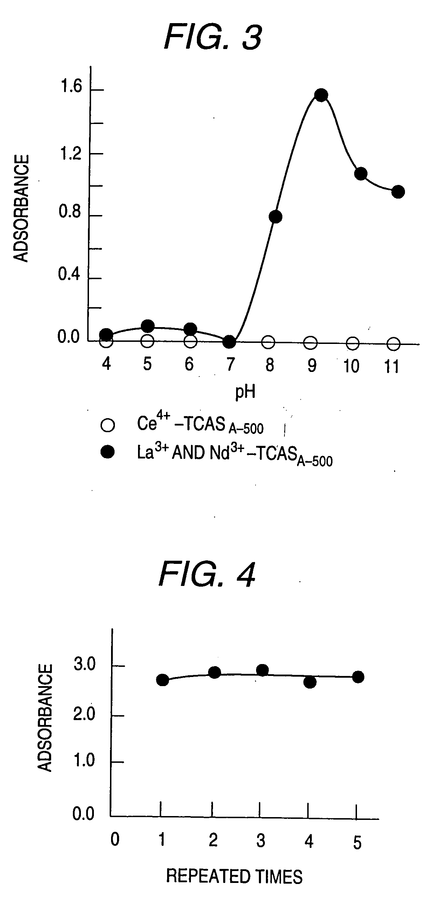

Fig. 3 is a graph showing the absorbance at 400 nm for each pH of the p-nitrophenolate (λmax: 400 nm) which is a hydrolyzate obtained by hydrolyzing sodium bis(p-nitrophenyl) phosphate (BNPP) at pH's of from 4 to 11 using the catalytic function of a cyclic phenol sulfide sulfonic acid cerium complex of the present invention.
Fig. 4 is a graph obtained by conducting the hydrolysis of BNPP using, as a catalyst, a cyclic phenol sulfide sulfonic acid cerium complex of the present invention, recovering and reusing the catalyst used to repeatedly conduct the hydrolysis five times, and determining the absorbance at 400 nm of the p-nitrophenolate (λmax: 400 nm) obtained in each hydrolysis.

### Best Mode for Carrying Out the Invention

The present invention will be explained below in detail.

In formula (1), X represents a hydrogen atom, a hydrocarbon group, an acyl group, a carboxyalkyl group, or a carbamoylalkyl group.

The carbon atom number of the hydrocarbon group of X is not particularly limited so long as it is 1 or more; 1 to 6. The hydrocarbon group may be saturated or unsaturated, or straight or branched. Specific examples include methyl, ethyl, n-propyl, isopropyl, n-butyl, n-pentyl, n-hexyl, vinyl, allyl, cyclohexyl, phenyl, and the like.

The carbon atom number of the acyl group of X is not particularly limited so long as it is 1 or more; however, it is preferably from 1 to 10, more preferably from 1 to 7. Preferred examples of the acyl group include formyl, acetyl, propionyl, butyryl, valeryl, oxalyl, malonyl, succinyl, benzoyl, acryloyl, methacryloyl, crotonyl, and the like.

The carbon atom number of the carboxyalkyl group of X is not particularly limited so long as it is 2 or more; however, it is preferably from 2 to 15, more preferably from 2 to 13. Preferred examples of the carboxyalkyl group include -CH₂COOH, -CH₂CH₂COOH, -CH(CH₃)COOH, -CH₂CH₂CH₂COOH, -C(CH₃)₂COOH, and the like.

The carbon atom number of the carbamoylalkyl group of X is not particularly limited so long as it is 2 or more; however, it is preferably from 2 to 15, more preferablly from 2 to 13. Preferred examples of the carbamoylalkyl group include -CH₂CONH₂, -CH₂CH₂CONH₂, -CH(CH₃)CONH₂, -C (CH₃)₂CONH₂, -CH₂CONH(CH₃), -CH₂CONHCH(CH₃)Ph (Ph represents a phenyl group), and the like.

The hydrogen atoms in these hydrocarbon group, acyl group, carboxyalkyl group or carbamoylalkyl group may be substituted with a functional group, such as a hydrocarbon group, a halogen atom, a nitro group, a carboxyl group, an acyl group, an amino group, an amido group, a hydroxyl group, or the like.

Also, when the carbon number of the hydrocarbon group, acyl group, carboxyalkyl group and carbamoylalkyl group of X increases, generally, a capability of forming a complex with a metal tends to be lowered.

In formula (1), the number of X's present is from 4 to 8 per molecule. The plural X's are the same or different. When the presence ratio of X other than a hydrogen atom in the plural X's is high, a capability of forming a complex with a metal may be lowered.

In formula (1), Y represents a hydrogen atom, a hydrocarbon group, a halogenated hydrocarbon group, a halogen atom, an acyl group, a hydroxyl group, a carboxyl group, an amido group, an amino group, a nitro group, a cyano group, a chlorosulfonic acid group, an alkoxysulfonyloxy group, or a sulfonic acid group.

The carbon atom number of the hydrocarbon group of Y is not particularly limited so long as the number is 1 or more; however, it is preferably from 1 to 30, more preferably from 1 to 18. Examples of the hydrocarbon group include a saturated aliphatic hydrocarbon group, an unsaturated aliphatic hydrocarbon group, an alicyclic hydrocarbon group, an alicyclic-aliphatic hydrocarbon group, an aromatic hydrocarbon group, an aromatic-aliphatic hydrocarbon group, and the like.

Preferred examples of the saturated aliphatic hydrocarbon group include an alkyl group, such as methyl, ethyl, n-propyl, isopropyl, n-butyl, isobutyl, *tert*-butyl, n-pentyl, neopentyl, n-hexyl, n-octyl, *tert*-octyl, n-nonyl, isononyl, n-dodecyl, and the like; a hydrocarbon group derived from a polymer or copolymer of ethylene, propylene, or butylene; and the like. The alkyl group may be straight, branched, or cyclic.

Preferred examples of the unsaturated aliphatic hydrocarbon group include alkenyl groups and alkynyl groups, such as vinyl, allyl, isopropenyl, 2-butenyl, and the like; a hydrocarbon group derived from a polymer or copolymer of acetylene, butadiene, or isoprene; and the like.

Preferred examples of the alicyclic hydrocarbon group include a cycloalkyl group, a cycloalkenyl group, a cycloalkynyl group, and the like, such as cyclohexyl, methylcyclohexyl, ethylcyclohexyl, and the like.

Preferred examples of the alicyclic-aliphatic hydrocarbon group include an alkyl group, an alkenyl group, an alkynyl group, and the like substituted with a cycloalkyl group, a cycloalkenyl group, or a cycloalkynyl group, such as cyclohexylmethyl, cyclohexylethyl, and the like.

Preferred examples of the aromatic hydrocarbon group include an aryl group, such as phenyl, naphthyl, and the like; an alkylaryl group, such as methylphenyl, dimethylphenyl, trimethylphenyl, ethylphenyl, butylphenyl, and the like; and the like.

Preferred examples of the aromatic-aliphatic hydrocarbon group include an aralkyl group and the like, such as benzyl, phenylethyl, phenylpropyl, phenylbutyl, methylphenylethyl, and the like.

Also, these hydrocarbon groups may be substituted with a functional group, such as a halogen atom, an acyl group, a hydroxyl group, a carboxyl group, an amido group, an amino group, a nitro group, or the like.

Furthermore, the halogenated hydrocarbon group include those similar to the hydrocarbon groups explained in the above Y, which are substituted with a halogen atom.

The halogen atom may be any atom of fluorine, chlorine, bromine and iodine.

The acyl group includes those similar to the acyl groups explained in the above X, and the preferred groups are also similar.

Examples of the amido group include 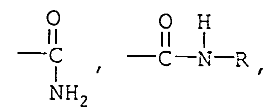 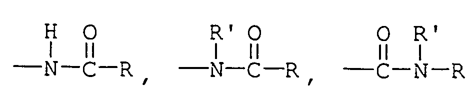 (R and R' each independently represents a hydrocarbon group, and examples of R and R' include the hydrocarbon groups explained in the above Y), and the like.

Examples of the alkyl moiety of the alkoxysulfonyloxy group include the alkyl groups exemplified in the hydrocarbon groups explained in the above Y.

A part or all of the hydrogen atoms constituting each group of the hydroxyl group, carboxyl group, amido group, and alkoxysulfonyloxy group may be substituted with a hydrocarbon group. The hydrocarbon group includes those similar to the hydrocarbon group explained in X, and the preferred groups are also the same.

Furthermore, the sulfonic acid group of Y in formula (1) is sulfonic acid (-SO₃H), or a metal salt, an ammonium salt, a (lower alkyl)ammonium salt, a (lower alkanol)ammonium salt or a pyridinium salt of sulfonic acid.

Examples of a metal in the sulfonic acid group include an alkali metal, such as sodium, potassium, and the like; an alkaline earth metal salt, such as calcium, magnesium, and the like; and the like.

The (lower alkyl)ammonium in the sulfonic acid group of Y preferably has an alkyl moiety of a carbon number of from 1 to 12, and examples thereof include methylammonium, ethylammonium, n-propylammonium, isopropylammonium, n-butylammonium, isobutylammonium, *sec*-butylammonium, *tert*-butylammonium, dimethylammonium, diethylammonium, di-n-propylammonium, diisopropylammonium, di-n-butylammonium, diisobutylammonium, di-*sec*-butylammonium, di-tert-butylammonium, trimethylammonium, triethylammonium, and the like.

The (lower alkanol)ammonium in the sulfonic acid group of Y preferably has an alkyl moiety of from 1 to 10, and examples thereof include ethanolammonium, diethanolammonium, triethanolammonium, and the like.

Examples of the pyridinium salt in the sulfonic acid group of Y include pyridinium, *N*-methylpyridinium, and the like.

In formula (1), the number of Y present is from 4 to 8 per molecule. The plural Y's are the same or different.

In formula (1), Z represents Sm, SO, or SO₂.

Sm is a polysulfide group, such as a sulfide group, a disulfide group, or the like, and m of Sm is an integer of from 1 to 7, and preferably 1 to 3. The plural m's of Sm are the same or different. Also, SO is a sulfinyl group, and SO₂ is a sulfonyl group.

In formula (1), the number of Z's present is from 4 to 8 per molecule. The plural Z's are the same or different. When the presence ratio of Z other than Sm in the plural Z's, that is, a sulfinyl group or a sulfonyl group, is high, a capability of forming a complex with a transition metal or a rare earth metal may be lowered.

In formula (1), n is an integer of from 4 to 8.

M in formula (1) is a metal of the Groups 8, 1A to 7A 1B or 3B.

Examples of the metal of the Group 8 include iron (Fe), cobalt (Co), nickel (Ni), ruthenium (Ru), rhodium (Rh), palladium (Pd), osmium (Os), iridium (Ir), and platinum (Pt).

Examples of alkali metal and alkaline earth metal of the Groups 1A and 2A include sodium (Na), potassium (K), rubidium (Rb), cesium (Cs), magnesium (Mg), calcium (Ca), barium (Ba), and the like.

Examples of the metal of the Groups 3A to 7A include titanium (Ti), vanadium (V), chromium (Cr), manganese (Mn), yttrium (Y), zirconium (Zr), niobium (Nb), molybdenum (Mo), hafnium (Hf), tantalum (Ta), tungsten (W) , rhenium (Re), and the like, in addition to rare earth metals, such as lanthanum (La), cerium (Ce), neodymium (Nd), samarium (Sm), europium (Eu), gadolinium (Gd), terbium (Tb), holmium (Ho), erbium (Er), thulium (Tm), ytterbium (Yb), lutetium (Lu), and the like.

Examples of the metal of the Groups 1B and 3B include copper (Cu), silver (Ag), gold (Au), aluminum (Al), gallium (Ga), indium (In), and thallium (Tl).

M in formula (1) is preferably a metal of the Groups 8, 3A to 7A or 1B.

p and q in formula (1) represent a composition ratio, and an integer of 1 or more. p and q change depending on the kind of the metal, the kind of the cyclic phenol sulfide, the kind of the solvent, and the like; however, in many cases the ratio is 1:1.

Next, the cyclic phenol sulfide metal complex of formula (3) is explained below.

The cyclic phenol sulfide metal complex of formula (3) is the same as the cyclic phenol sulfide metal complex of formula (1), except that M is replaced with M¹. Accordingly, X, Y, Z, m, n, p and q in formula (3) are the same as those of X, Y, Z, m, n, p and q in formula (1) in content and condition, and preferred examples are also the same.

M¹ in formula (3) represents a transition metal or a rare earth metal.

Preferred examples of the transition metal and rare earth metal include iron (Fe), cobalt (Co), nickel (Ni), ruthenium (Ru), rhodium (Rh), palladium (Pd), osmium (Os), iridium (Ir), and platinum (Pt) of the Group 8; titanium (Ti), vanadium (V), chromium (Cr), manganese (Mn), copper (Cu), zinc (Zn), yttrium (Y) , zirconium (Zr) , niobium (Nb), molybdenum (Mo), silver (Ag), cadmium (Cd), lanthanum (La), cerium (Ce), neodymium (Nd), samarium (Sm), europium (Er), thulium (Tm), ytterbium (Yb), lutetium (Lu), hafnium (Hf), tantalum (Ta), tungsten (W), rhenium (Re), gold (Au), and mercury (Hg) of the Groups 3A to 7A, 1B and 2B; and the like.

A process for producing the cyclic phenol sulfide metal complexes represented by formulae (1) and (3) is explained below.

The cyclic phenol sulfide metal complexes represented by formulae (1) and (3) are each obtained by bringing a cyclic phenol sulfide represented by formula (2) into contact with a metal.

A process for producing a cyclic phenol sulfide represented by formula (2) is described in JP-A-9-227553 and WO 98/09959. An appropriate process is a process comprising first reacting alkylphenol represented by formula (4) having a hydrocarbon group at the 4-position: 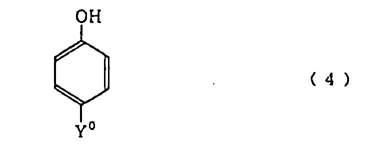 (in formula (4), Y⁰ represents a hydrocarbon group) with an appropriate amount of elemental sulfur in the presence of an appropriate amount of at least one metallic reagent selected from an alkali metal reagent and an alkaline earth metal reagent.

The phenol compound and elemental sulfur used as starting materials are fed in such a proportion that the amount of the elemental sulfur is 0.1 gram equivalent or more, preferably 0.35 gram equivalents or more, per gram equivalent of the phenol compound. Although there is no particular upper limit on the proportion of the elemental sulfur fed as a starting material, the amount thereof is preferably 20 gram equivalents or less, particularly preferably 10 gram equivalents or less, per gram equivalent of the phenol compound.

Examples of the alkali metal reagent include an elemental alkali metal, an alkali metal hydride, an alkali metal hydroxide, an alkali metal carbonate, an alkali metal alkoxide, an alkali metal halide, and the like. Examples of the alkaline earth metal reagent include an elemental alkaline earth metal, an alkaline earth metal hydride, an alkaline earth metal hydroxide, an alkaline earth metal oxide, an alkaline earth metal carbonate, an alkaline earth metal alkoxide, an alkaline earth metal halide, and the like.

The alkali metal reagent or the alkaline earth metal reagent is used at an amount of 0.005 gram equivalents or more, preferably 0.01 gram equivalent or more, per gram equivalent of the phenol compound. Although there is no particular upper limit on the amount used of the alkali metal reagent or the alkaline earth metal reagent, the amount thereof is preferably 10 gram equivalents or less, and more preferably 5 gram equivalents or less.

The hydrogen atoms of the phenolic hydroxyl groups of the cyclic phenol sulfide intermediate yielded can be converted, if desired, to X¹ in formula (2) other than a hydrogen atom.

Examples of this conversion method are described in JP-A-9-227553. Appropriate production examples include a method in which the phenolic hydroxyl groups of the cyclic phenol sulfide intermediate are acylated with an acylating agent such as acetyl chloride. The examples further include a method in which the hydrogen atoms of the phenolic hydroxyl groups of the cyclic phenol sulfide intermediate are replaced with an alkali metal, which is then converted to a hydrocarbon group by reaction with a halogenated hydrocarbon.

Furthermore, the hydrocarbon groups in the p-position to the phenolic hydroxyl groups of the cyclic phenol sulfide intermediate yielded can be converted to a hydrogen atom if desired.

Examples of this conversion method are described in JP-A-10-77282. Appropriate production examples include a method in which the hydrocarbon groups in the p-position to the phenolic hydroxyl groups of the cyclic phenol sulfide intermediate are subjected to hydrocarbon elimination with a catalyst, such as aluminum chloride or the like.

Examples of methods for conversion into Y¹ in formula (2) other than a hydrogen atom and a hydrocarbon group are described in JP-A-9-227553. Appropriate production examples include a method in which an appropriate nitrating agent such as nitric acid is caused to act on the cyclic phenol sulfide represented by formula (2) wherein Y¹ is a hydrogen atom to thereby convert into a nitro group.

The nitro groups can be converted to amino groups by reducing them with an appropriate reducing agent such as iron/hydrochloric acid. Furthermore, by diazotizing these amino groups with sodium nitrite or the like and then subjecting the diazotized groups to the action of an appropriate halogenating agent, such as copper chloride or the like, or of water in the presence of hydrochloric acid or the like, the amino groups can be converted to halogen groups or hydroxyl groups, respectively.

Examples thereof further include a method in which the cyclic phenol sulfide which has undergone the hydrocarbon elimination is reacted with an acid halide, if necessary, in the presence of a catalyst, such as a Lewis acid or the like, to thereby convert to acyl groups.

Furthermore, the hydrocarbon groups in the p-position to the phenolic hydroxyl groups of the cyclic phenol sulfide intermediate yielded can be converted to sulfo groups. Examples of methods for this conversion are described in the description of WO 99/29683. An appropriate production example is a method in which the cyclic phenol sulfide intermediate is suspended in sulfuric acid and stirred therein under heating.

The concentration of sulfuric acid used in this reaction may be 80% or higher, preferably 90% or higher.

Although the use amount of sulfuric acid is not particularly limited, it may usually be from 5 to 200 ml per g of the cyclic alkylphenol sulfide.

The reaction temperature is preferably 70°C or higher, more preferably 80°C or higher.

Although the reaction time is not particularly limited, it may usually be from 2 hours to 30 hours.

After completion of the reaction, the reaction product is diluted with water and subjected to salting out with a metal salt, such as an alkali metal, alkaline earth metal salt, or the like, an ammonium salt, a (lower alkyl)ammonium salt, a (lower alkanol)ammonium salt, or a pyridinium salt, to obtain a cyclic phenol sulfide represented by formula (2) wherein Y¹ is a sulfo group.

Furthermore, the sulfide groups of the cyclic phenol sulfide can be converted to Z¹ in formula (2) other than a sulfide group.

Examples of methods for this conversion are described in WO 98/09959. Appropriate production examples include a method in which the sulfide groups are oxidized with an oxidizing agent, such as hydrogen peroxide, sodium perborate, or the like, to thereby convert these groups into sulfinyl groups or sulfonyl groups.

Given metals are caused to act on the thus-obtained cyclic phenol sulfide represented by formula (2), whereby a cyclic phenol sulfide metal complex represented by formula (1) or formula (3) can be produced.

Examples of methods for causing a metal to act on the cyclic phenol sulfide represented by formula (2) include a method in which the cyclic phenol sulfide is brought into contact with the metal. Appropriate examples of contacting methods include a method in which a solution containing the cyclic phenol sulfide dissolved therein and a solution containing the metal dissolved therein are contacted with each other by stirring, shaking, refluxing with heating, or the like.

Some cyclic phenol sulfides represented by formula (2) are water-insoluble and the others are water-soluble. Examples of the solvent in which the cyclic phenol sulfides are to be dissolved include water-insoluble solvents for the cyclic phenol sulfides which are water-insoluble and water-soluble solvents for the cyclic phenol sulfides which are water-soluble.

Appropriate specific examples of the water-insoluble solvents include aromatic hydrocarbons, such as benzene, toluene, xylene, and the like; halogenated hydrocarbons, such as chloroform, carbon tetrachloride, chlorobenzene, and the like; aliphatic hydrocarbons, such as hexane, octane, and the like; and mineral oils, such as kerosine, gas oil, and the like.

Appropriate specific examples of the water-soluble solvents include water, alcohols, such as ethanol and the like, pyridine, acetone, and the like.

Solvents may be used alone or as a mixture of two or more thereof.

The concentration of the cyclic phenol sulfide represented by formula (2) in solvents is not particularly limited, except that the upper limit thereof is limited by the solubility of the cyclic phenol sulfide in each solvent. Generally, the concentration thereof is from 1×10⁻⁶ to 1 M.

The metal which is caused to act on the cyclic phenol sulfide represented by formula (2) in order to produce a cyclic phenol sulfide metal complex represented by formula (1) is a metal of the Groups 8, 1A to 7A, 1B, or 3B. Specific examples thereof include those similar to the metals exemplified above in M in formula (1).

Metal ions (metal salts) during complex formation may be present in any amount so long as the number thereof is substantially not smaller than the number of molecules of the sulfide of formula (2). However, from the standpoint of a balance between the efficiency of reaction operation and profitability, metal ions are usually used in an amount of from 1.2 to 100 times by mole the amount of the sulfide. In the case of using a large excess of metal ions (metal salts), an economical burden can be reduced by recovering and reusing the aqueous solution resulting from the complex formation treatment. In the case of using a water-soluble sulfide, it is preferred to conduct complex formation in a homogeneous aqueous solution system; this operation can be performed, for example, by adding an aqueous metal salt solution to an aqueous solution of the sulfide. In this case, use of a large excess of the metal is disadvantageous because of the necessity of separating the metal ions (metal salts) which have not participated in complex formation. Usually, the aqueous metal ion (metal salts) solution is added in an amount of from 1.2 to 5 times the amount of the aqueous sulfide solution. Although the unreacted or excess metal ions (metal salt) remain in the solution in this method, these may be removed with an ion-exchange resin or the like.

Moreover, the metal which is caused to act on the cyclic phenol sulfide represented by formula (2) in order to produce a cyclic phenol sulfide metal complex represented by formula (3) is a transition metal or a rare earth metal. Specific examples thereof include those similar to the metals exemplified above in M¹ in formula (3).

Furthermore, conditions and the like for producing a cyclic phenol sulfide metal complex represented by formula (3) are the same as in the production of a cyclic phenol sulfide metal complex represented by formula (1) described above.

Various metal compounds can be used as the metal to be caused to act on the cyclic phenol sulfide represented by formula (2).

Preferred examples of the metal compounds include metal salts, such as halides, nitric acid salts, and salts with organic acids (e.g., picric acid *etc.),* and the like. However, coordination compounds with organic ligands, such as carbonyl, acetylacetone, and the like, can also be used.

The solvent in which the metal is to be dissolved varies depending on the kind of the metal compound. Namely, examples thereof include water-soluble solvents for metal salts and water-insoluble solvents for organic coordination compounds. With respect to each of the water-soluble solvents and the water-insoluble solvents, examples thereof include those similar to the solvents exemplified above, and preferred examples thereof are also similar.

The concentration of the metal dissolved in a solvent is not particularly limited, and complex formation can be sufficiently accomplished even at about 1×10⁻⁶ M. Heightening the metal concentration arouses no troubles, and the concentration thereof can be determined while taking account of the molar ratio thereof to the sulfide to be contacted therewith, the volume ratio to the liquid layer to be contacted therewith in the case of a two-layer system and the like. However, the upper limits are the solubilities of the metal and sulfide used, because recovery of the excess metal requires much labor in the case of using a large excess of the metal in a homogeneous system as stated above, and from the standpoint of the efficiency of liquid-liquid contact in the case of a two-layer system.

In the case where the solvent for the metal is water, the aqueous metal solution is not particularly limited in pH. However, the pH is preferably from 3 to 11. As the pH decreases to below 3, the metal, in complex formation with the cyclic phenol sulfide, tends to have a lower degree of complex formation and comes to necessitate a longer time period for contact with the cyclic phenol sulfide.

The operation for complex formation from the cyclic phenol sulfide and the metal is conducted by bringing a solution containing the cyclic phenol sulfide dissolved therein and a solution containing the metal dissolved therein into contact with each other by stirring, shaking, refluxing with heating, or the like.

The reaction temperature for complex formation is not particularly limited, and the formation can be conducted at any temperature higher than the solidifying point of the solvent used and not higher than the boiling point thereof. However, too low temperatures may cause precipitation because of the reduced solubility of the sulfide. From the standpoint of utilizing the stirring effect of refluxing with heating, it is advantageous to conduct the reaction at around the boiling point.

Conditions for the stirring or shaking are not particularly limited. However, it may usually be conducted for from 1 to 72 hours. In general, vigorous shaking or stirring makes the complex formation more efficient. The shaking may usually be conducted at a frequency of about from 100 to 400 times per minute.

In the case where the cyclic phenol sulfide is water-insoluble and the solvent containing it dissolved therein is water-insoluble, this solution is brought into contact with a solution prepared by dissolving the metal in a water-soluble solvent so that the cyclic phenol sulfide metal complex yielded moves to the water-insoluble solvent.

Also, in the case where the cyclic phenol sulfide is water-soluble, the solvent in which the cyclic phenol sulfide is to be dissolved can be the same as the solvent in which the metal is to be dissolved.

Furthermore, in the case where the metal is in the form of a coordination compound with an organic substance, it can be added to the cyclic phenol sulfide and a water-insoluble solvent to conduct refluxing with heating.

Conditions for the refluxing with heating are not particularly limited as long as the temperature is not higher than the boiling point of the solvent. The refluxing may usually be conducted for from 1 to 72 hours.

From the thus-obtained solution containing a complex of the cyclic phenol sulfide with the metal, the water-insoluble solvent or water-soluble solvent is removed by a known technique such as distillation. Thus, each cyclic phenol sulfide metal complex of the present invention can be isolated.

Moreover, the cyclic phenol sulfide metal complexes isolated can be recrystallized if desired. The recrystallization can be conducted by a known technique using a solvent, such as ethyl acetate, dichloromethane, chloroform, dioxane, toluene, or the like, or a mixed solvent such as chloroform/acetone, benzene/methanol, toluene/ethanol, or the like.

The cyclic phenol sulfide metal complexes of the present invention can be further mixed with or supported on a solid support if desired.

When a cyclic phenol sulfide metal complex having a catalytic activity in oxidations with hydrogen peroxide is used in an analysis for hydrogen peroxide, the cyclic phenol sulfide metal complex can be used as it is or after having been dissolved in a solution. However, it is convenient and advantageous to use the complex which has been mixed with or supported on a solid support.

The cyclic phenol sulfide metal complex represented by formula (1) wherein Y is a sulfonic acid group is water-soluble. Examples of the solvent in which this cyclic phenol sulfide is to be dissolved include water-soluble solvents. Preferred examples of the water-soluble solvents include water.

Examples of the solid support include silica gel, ion-exchange resins, ion exchangers, glasses, carbon, diatomaceous earth, cellulose, chitosan, and the like. Although the solid support is not particularly limited, preferred examples thereof are weakly basic anion-exchange resins and anion exchangers, cellulose, chitosan, and the like.

The contact operation is conducted by mixing a solution containing the cyclic phenol sulfide metal complex dissolved therein with the solid support by shaking, stirring, or the like. Conditions for the shaking or stirring are not particularly limited. Mixing may be followed by standing.

The temperature at which the complex is contacted with the solid support or with a solution containing the same is not particularly limited, as long as it is not higher than the boiling point of the solvent used. Usually, the operation may be conducted at a temperature around room temperature, i.e., from 5°C to 35°C.

Incidentally, a technique which may be used for mixing or supporting a cyclic phenol sulfide metal complex with or on a solid support comprises producing the cyclic phenol sulfide metal complex beforehand and then contacting it with the solid support, as described above. However, use may be made of a method comprising mixing or supporting a cyclic phenol sulfide with or on a solid support beforehand in the same manner and then causing a metal to act thereon to conduct complex formation with the metal.

In the case where a cyclic phenol sulfide metal complex is used after having been mixed with or supported on a solid support, the content of the cyclic phenol sulfide metal complex is not particularly limited. However, the content thereof is preferably from 1 to 500 µmol, more preferably from 20 to 200 µmol, per g of the support.

The cyclic phenol sulfide metal complex represented by formula (1) can be used in applications such as catalysts for various chemical reactions, information/electronic materials, and the like.

By using either the cyclic phenol sulfide metal complex represented by formula (3) or the cyclic phenol sulfide metal complex mixed with or supported on a solid support (hereinafter often referred to as the "complex of the present invention"), it is possible to use it as a catalyst for oxidation by hydrogen peroxide and to analyze hydrogen peroxide.

When the complex of the present invention is used as a catalyst for oxidation by hydrogen peroxide, an oxidizable substance is reacted with hydrogen peroxide in the presence of the compound of the present invention. The oxidizable substance used in an oxidation with hydrogen peroxide is not particularly limited so long as it is oxidizable by hydrogen peroxide. Examples thereof include substances causative of environmental variation and the like, such as IQ (2-amino-3-methylimidazo[4,5-f]quinoline), MeIQ (2-amino-3,4-dimethylimidazo[4,5-f]quinoline), MeIQ (2-amino-3,8-dimethylimidazo[4,5-f]quinoxaline), TrpP-1 (3-amino-1,4-dimethylpyrido[4,3-b]indole), TrpP-2 (3-amino-1-methylpyrido[4,3-b]indole), and the like.

The use amount of the complex of the present invention in oxidations with hydrogen peroxide is not particularly limited. However, too small amounts may result in a considerably reduced reaction rate, while too large amounts may be disadvantageous from an economical standpoint. Consequently, it is preferably from 0.01 to 35 mol, more preferably from 0.1 to 15 mol, per mol of the hydrogen peroxide.

Quantitative analysis for hydrogen peroxide is conducted by reacting an oxidizable substance with hydrogen peroxide in the presence of the complex of the present invention and directly or indirectly determining the amount of a reaction product. For example, a calibration curve obtained from changes of the amount of the reaction product, determined by utilizing spectroscopy, fluorescence, radiation, or the like, and from the amount of hydrogen peroxide can be used to detect/determine hydrogen peroxide. More specifically, hydrogen peroxide, 4-aminoantipyrine (4-AAP), and phenol are reacted in the presence of the complex of the present invention, and the absorbance of the quinoid type dye yielded is determined at 505 nm. The linear relationship between the increase in absorbance and the amount of hydrogen peroxide is utilized to determine hydrogen peroxide.

Furthermore, by using either the cyclic phenol sulfide metal complex represented by formula (3) or the cyclic phenol sulfide metal complex mixed with or supported on a solid support, it is possible to use it as a catalyst for hydrolysis of phosphodiester.

When the complex of the present invention is used as a catalyst for hydrolysis of phosphodiester, the phosphodiester hydrolysis is conducted in the presence of the compound of the present invention. Examples of samples used in the phosphodiester hydrolysis include various phosphodiester, and examples include nucleic-acid-related compounds, such as DNA and RNA; phospholipids, such as 3-phosphatidylcholine, 3-phosphatidylethanolamine, 3-phosphatidylserine, 3-phosphatidylethanolamine, 1-alkoxyphospholipids, 3-phosphatidylinositol, and 3-phosphatidylglycerol, and the like; and the like.

The use amount of the support of the present invention in the hydrolysis of phosphodiester is not particularly limited. However, too small amounts may result in a considerably reduced reaction rate, while too large amounts may be disadvantageous from an economical standpoint. Consequently, it is preferably from 0.05 to 150 mol, more preferably from 0.5 to 70 mol, per mol of the phosphodiester.

The present invention will be explained in more detail by means of Production Examples and Examples. However, the present invention should not be construed as being limited by these examples in any way. In the following Production Examples and Examples, "t-Bu" represents a tert-butyl group.

### Production Example 1

### Synthesis of 5,11,17,23-tetra-tert-butyl-25,26,27,28-tetrahydroxy-2,8,14,20-tetrathia [19.3.1.1^{3,7}1^{9,13}1^{15,19}]octacosa-1(25),3,5,7(28),9,11,13(27),15,17,19(26),21,23-dodecaene (I):

To 45.2 g of 4-tert-butylphenol, 14.4 g elemental sulfur and 3.0 g of sodium hydroxide were added, followed by heating up to 230°C over 4 hours under stirring in an atmosphere of nitrogen, and then stirring for further 2 hours During the reaction, water and a hydrogen sulfide generated by the reaction were eliminated. The water distilled during the reaction was about 0.8 g, and the hydrogen sulfide formed by the reaction was about 6 g. This reaction mixture was cooled to room temperature, dissolved in 500 ml of ether added, and hydrolyzed with a 1 N aqueous sulfuric acid solution. A separated ether layer was washed with water and dried with magnesium sulfate. The reaction mixture obtained after evaporation of ether was further partitioned by silica gel column chromatography (hexane/chloroform) to give a crude product. The crude product was recrystallized from chloroform/acetone to give 4.32 g of Product (I) as colorless transparent crystals.

This Product (I) is a cyclic alkylphenol sulfide of formula (2) in which X¹ = H, Y¹ = *t*-Bu, Z¹ = S (m¹ = 1), and n¹ = 4.

Physical properties are shown below.
Melting point: 320-322°C
¹H-NMR: (δ, ppm, CDCl₃) 9.60 (s, 4H, OH), 7.64 (s, 8H, ArH), 1.22 (s, 36H, C(CH₃)₃)
¹³C-NMR: (δ, ppm, CDC₁₃) 155.6, 144.7, 136.4, 120.5 (Ar), 34.2 (C(CH₃)), 31.3(C(CH₃)₃)
IR: (cm⁻¹, KRS-5) 3324 (OH), 2962 (CH)
MS m/z: 720 (M⁺)

| Elemental analysis (%): | | | |
|---|---|---|---|
| Calculated for C₄₀H₄₈S₄O₄ | C: 66.62 | H: 6.71 | S: 17.79 |
| Found | C, 66.37 | H, 6.57 | S, 17.22 |

### Production Example 2

### Synthesis of sodium 25,26,27,28-Tetrahydroxy-2,8,14,20-tetrathia[19.3.1.1^{3,7}1^{9,13}1^{15,19}]octacosa-1(25),3,5,7(28),9,11,13(27),15,17,19(26),21,23-dodecaene-5,11,17,23-tetrasulfonate (II):

In 15 ml of concentrated sulfuric acid was suspended 152 mg of the cyclic phenol sulfide (I) obtained in Production Example 1, heated up to 80°C and allowed to react for 4 hours. After this reaction liquid was allowed to cool and then diluted to 100 ml with purified water, the unreacted cyclic phenol sulfide was filtered off. Thereafter, sodium chloride was added, followed by salting out to obtain 203 mg of a white powder. Salting out was further conducted several times to obtain 151 mg of Product (II).

This Product (II) is a cyclic phenol sulfide represented by formula (2) in which X¹ = H, Y¹ = SO₃Na, m¹ = 1, and n¹ = 4.

Physical properties are shown below.
Melting point: 370-390°C (decomposition point)
¹H-NMR: (δ, ppm, 25 mg/0.6 ml D₂O) 8.87 (s, 8H, ArH)
¹³C-NMR: (δ, ppm, 25 mg/0.6 ml D₂O) 139.30, 136.89, 129.08, 124.79 (Ar)
FAB-MS (m/z) : 905 (M⁺+1)

| Elemental analysis (%) : | | | | |
|---|---|---|---|---|
| Calculated for C₂₄H₁₂S₈O₁₆Na₄ | C: 31.46 | H: 2.83 | S:28.35 | Na: 10.16 |
| Found | C: 31.2 | H: 3.1 | S: 28.7 | Na: 10.1 |

### Production Example 3

### Synthesis of 5,11,17,23-Tetra-tert-butyl-25,26,27,28-tetrahydroxy-2,8,14,20-tetrasulfinyl [19.3.1.1^{3,7}1^{9,13}1^{15,19}]-octacosa-1(25),3,5,7(28),9,11,13(27) ,15,17,19(26),21,23-dodecaene (III) :

In 30 ml of chloroform was dissolved 1.8 g of the cyclic phenol sulfide (I) obtained in Production Example 1. A solution prepared beforehand by dissolving 5.7 g of 30% aqueous hydrogen peroxide solution in 100 ml of glacial acetic acid was added dropwise to the chloroform solution at room temperature over 30 minutes, followed by stirring at room temperature for 24 hours. To the reaction solution obtained, 150 ml of water was added. This mixture was extracted with chloroform (50 ml × 3), and the chloroform layer was washed with water. After the layer was dried with anhydrous magnesium sulfate, the solvent was distilled off to obtain 522 mg of a white powder, which was sufficiently washed with methanol. Thus, 485 g of Product (III) was obtained.

This Product (III) is a cyclic phenol sulfinyl compound represented by formula (2) wherein X¹ = H, Y¹ = t-Bu, Z¹ = sulfinyl, and n¹ = 4.

Physical properties are shown below.
Melting point: 210°C (decomposition point)
¹H-NMR: (δ, ppm, Cl₂CDCDCl₂) 9.20 (s, 4H, OH), 7.61 (s, 8H, ArH), 1.26 (s, 36H, C(CH₃)₃)
¹³C-NMR: (δ, ppm, Cl₂CDCDCl₂) 152.7, 142.4, 130.2, 128.0, 124.2, 122.8 (Ar), 34.8 (C(CH₃)₃), 31.4 (C(CH₃)₃) FT-IR: (cm⁻¹, KBr) 3074 (br, OH), 2960 (s, CH), 1051, 998 (s, SO)
MS: (m/z) 785 (M⁺+1)

| Elemental analysis (%) : | | | |
|---|---|---|---|
| Calculated for C₄₀H₄₈S₄O₈ | C: 61.20 | H: 6.16 | S: 16.34 |
| Found | C: 61.1 | H: 6.3 | S: 15.9 |

### Example 1

Production of complex of cyclic phenol sulfide (I) obtainec in Production Example 1 (represented by formula (2) in which X¹ = H, Y¹ = t-Bu, Z¹ = S (m¹ = 1), and n¹ = 4) with metal:

In a 30-ml screw vial, 10 ml of a solution prepared by dissolving the cyclic phenol sulfide (I) in chloroform in a concentration of 1×10⁻³ M and 10 ml of an aqueous solution prepared by dissolving each of MnCl₂, FeCl₂, and CoCl₂ in water in a concentration of 1×10⁻³ M (adjusted to pH = 8.0 with Tris-HCl) were placed, followed by shaking at room temperature for 24 hours. After the mixture was allowed to stand, the chloroform layer was separated from the aqueous layer, and the chloroform was evaporated. The products were a white powder, a purple powder, and a yellow-green powder, respectively.

The complexes obtained are cyclic phenol sulfide metal complexes (IV), (V), and (VI) represented by formula (1) in which X = H, Y = t-Bu, Z = S (m = 1), n = 4, p = 1, q = 1, and M is Mn, Fe, and Co, respectively.

Physical properties are shown below.
Cyclic phenol sulfide manganese complex (IV):
UV-VIS (CHCl₃): λₛₕ317 nm (ε10100), λ307 nm (ε11500), λ297 nm (ε11500), λ242 nm (ε19100)

| Elemental analysis (%): | | | | |
|---|---|---|---|---|
| Calculated for C₄₀H₄₈S₄O₄Mn | C: 62.1 | H: 6.0 | S: 16.6 | Mn: 7.1 |
| Found | C: 61.1 | H: 6.0 | S: 15.9 | Mn: 6.8 |

Cyclic phenol sulfide iron complex (V):
UV-VIS (CHCl₃): λ500 nm (ε278), λₛₕ302 nm (ε15100), λ296 nm (ε19000)

| Elemental analysis (%) | | | | |
|---|---|---|---|---|
| Calculated for C₄₀H₄₈S₄O₄Fe | C: 62.0 | H: 6.0 | S: 16.5 | Fe: 7.2 |
| Found | C: 61.3 | H: 6.1 | S: 16.1 | Fe: 7.0 |

Cyclic phenol sulfide cobalt complex (VI):
UV-VIS (CHCl₃): λ307 nm (ε14300) λₛₕ295 nm (ε11500), λ244 nm (ε19900)

| Elemental analysis (%) | | | | |
|---|---|---|---|---|
| Calculated for C₄₀H₄₈S₄O₄Co | C: 61.8 | H: 5.9 | S: 16.5 | Co: 7.6 |
| Found | C: 61.0 | H: 5.9 | S: 15.9 | Co: 8.0 |

### Example 2

### Production of complex of cyclic phenol sulfide (I) obtained in Production Example 1 (represented by formula (2) in which X¹ = H, Y¹ = t-Bu, Z¹ = S (m¹ = 1), and n¹ = 4) with metal:

In 30 ml of toluene, 1.0 g of the cyclic phenol sulfide (I) was dissolved, and 40 ml of a solution prepared by dissolving 1.49 g of bis (bis(trimethylsilyl)amido)cobalt in toluene/hexane was added thereto, followed by refluxing with heating. At the time when a half of the solvent had been evaporated, 20 ml of tetrahydrofuran was added thereto, followed by cooling. The product was yellow-green columnar crystals.

The complex obtained was a cyclic phenol sulfide metal complex (VI) represented by formula (1) in which X = H, Y = t-Bu, Z = S (m = 1), n = 4, p = 1, q = 1, and M = Co.

### Example 3

### Production of complex of cyclic phenol sulfinyl compound (III) obtained in Production Example 3 (represented by formula (2) in which X¹ = H, Y¹ = t-Bu, Z¹ = SO (sulfinyl), and n = 4) with metal:

In 2 ml of chloroform, 50 mg of the cyclic phenol sulfinyl compound (III) was dissolved. Each of an aqueous MgSO₄ solution (prepared by dissolving 7.7 mg in 1 ml of water and adjusting the pH to 8.0 with Tris-HCl) and an aqueous Ba(OH)₂ solution (prepared by dissolving 11 mg in 2 ml of water) was added thereto, followed by shaking at room temperature for 3 hours. The precipitates were taken out by filtration and dried. As a result, 16.8 mg of a white powder and 34.8 mg of another white powder were obtained as products.

The complexes obtained were cyclic phenol sulfinyl compound metal complexes (VII) and (VIII) represented by formula (1) in which X = H, Y = t-Bu, Z = SO (sulfinyl), n = 4, p = 1, q = 1, and M is Mg and Ba, respectively.

Physical properties are shown below.
Cyclic phenol sulfinyl compound magnesium complex (VII):

| Elemental analysis (%): | | | | |
|---|---|---|---|---|
| Calculated for C₄₀H₄₈S₄O₈Mg | C: 59.5 | H: 5.7 | S: 15.9 | Mg; 3.0 |
| Found | C: 58.4 | H: 5.5 | S: 15.7 | Mg; 2.8 |

Cyclic phenol sulfinyl compound barium complex (VIII):

| Elemental analysis (%): | | | | |
|---|---|---|---|---|
| Calculated for C₄₀H₄₈S₄O₈Ba | C: 52.2 | H: 5.0 | S: 13.9 | Ba; 14.9 |
| Found | C: 51.4 | H: 4.9 | S: 13.3 | Ba; 14.0 |

### Example 4

### Production of complex of sodium cyclic phenol sulfide sulfonate (II) obtained in Production Example 2 (represented by formula (2) wherein X¹ = H, Y¹ = SC₃Na, m¹ = 1, and n¹ = 4) with metal and of ion exchanger having the same:

To an aqueous solution prepared by dissolving 45.2 mg of the sodium cyclic phenol sulfide sulfonate (II) in 50 ml of water, 10 ml of an aqueous solution prepared by dissolving Ee(NH₄)₂(SO₄)₂·6H₂O in a concentration of 1.0×10⁻² M was gradually added dropwise under stirring, followed by further stirring for 1 hour. The unreacted iron ions were removed by adding 200 mg of a cation-exchange resin (Dowex MSC-1, manufactured by Muromachi Kagaku Kogyo Kaisha, Ltd.) to the reaction solution, followed by further stirring for 1 hour, and filtering off the cation-exchange resin.

The complex obtained is a cyclic phenol sulfide sulfonic acid iron complex (IX) represented by formula (3) in which X = H, Y = SO₃Na, m = 1, n = 4, p = 1, q = 1, and M¹ = Fe.

To a solution prepared by suspending 500 mg of an anion exchanger (DEAE cellulofine-sf, manufactured by Seikagaku Corporation) in 20 ml of water, 60 ml of an aqueous solution of the cyclic phenol sulfide sulfonic acid iron complex (IX) described above was gradually added dropwise, followed by stirring for 1 hour.

The ion exchanger obtained was filtered off with a membrane filter, sufficiently washed with water, and then dried *in vacuo* over P₂O₅.

Although the original ion exchanger was white, the cyclic phenol sulfide sulfonic acid iron complex (IX)/ion exchanger obtained was reddish-brown. The amount of the iron complex (IX) supported was 1×10⁻⁴ mol per g of the anion exchanger.

### Example 5

### Production of ion exchanger having cyclic phenol sulfide sulfonic acid iron complex (IV) by preparing ion exchanger having cyclic phenol sulfide sulfonic acid beforehand and then incorporating iron ion thereinto:

An aqueous solution prepared by dissolving 45.2 mg of the sodium cyclic phenol sulfide sulfonate (II) obtained in Production Example 2 in 50 ml of water was gradually added dropwise, under stirring, to a solution prepared by suspending 500 mg of an anion exchanger (DEAE cellulofine-sf, manufactured by Seikagaku Corporation) in 20 ml of water, followed by further stirring for 1 hour. The ion exchanger obtained was filtered off with a membrane filter, sufficiently washed with water, and then dried in *vacuo* over P₂O₅.

Subsequently, 10 ml of an aqueous solution prepared by dissolving Fe(NH₄)₂(SO₄)₂·6H₂O in a concentration of 1.0×10⁻² M was gradually added dropwise, under stirring, to a solution prepared by suspending 100 mg of the ion exchanger described above in 20 ml of water, followed by further stirring for 1 hour. The thus-obtained anion exchanger having a cyclic phenol sulfide sulfonic acid iron complex was filtered off with a membrane filter, sufficiently washed with water, and then dried *in vacuo* with P₂O₅.

It was reddish-brown like the cyclic phenol sulfide sulfonic acid iron complex (IX)/ion exchanger prepared in Example 4.

### Example 6

### Evaluation of anion exchanger having cyclic phenol sulfide sulfonic acid iron complex (IX) for catalytic activity in oxidation with hydrogen peroxide:

To a mixed solution consisting of 1 ml of a solution containing 35 µg of hydrogen peroxide, 1 ml of a 1 mg/ml solution of 4-AAP, 1 ml of a 5 mg/ml phenol solution, and 3 ml of a pH buffer, 20 ml of the anion exchanger obtained in Example 5 having a cyclic phenol sulfide sulfonic acid iron complex (IX) was added, followed by shaking at room temperature for 1 hour. The pH range was from 3 to 11. The buffer was prepared using 0.1 M sodium citrate and 0.1 M HCl for pH's of from 3 to 4, one prepared using 0.1 M KH₂PO₄ and 0.05 M Na₂B₄O₇ for pH ranges of from 5 to 9, or prepared using 0.05 M Na₂B₄O₇ and 0.05 M Na₂CO₃ for pH ranges of from 10 to 11. Subsequently, the ion exchanger having a cyclic phenol sulfide sulfonic acid iron complex was filtered out of the reaction liquid with a membrane filter. Thereafter, the solution obtained was examined for absorbance at 505 nm.

The results are shown in Fig. 1.

As a result, absorption at 505 nm was observed, which indicated that a quinoid type dye had been yielded from 4-AAP and phenol by the action of hydrogen peroxide in the presence of the anion exchanger having a cyclic phenol sulfide sulfonic acid iron complex (IX). The absorption was maximum at around pH = 10.

### Example 7

### Quantitative analysis for hydrogen peroxide:

At hydrogen peroxide concentrations of from 0 to 100 µg/ml and pH = 10, coloring by a quinoid type dye was examined in terms of absorption at 505 nm in the same manner as in Example 6.

The results are shown in Fig. 2.

There is a linear relationship between the increase in absorbance and the amount of hydrogen peroxide. Consequently, a quantitative analysis for hydrogen peroxide can be conducted by determining the absorbance at 505 nm and thereby determining the amount of the quinoid type dye yielded by the reaction of hydrogen peroxide, 4-AAP, and phenol by the catalytic action of the cyclic phenol sulfide sulfonic acid iron complex (IX).

### Example 8

### Evaluation of reproducibility of catalytic activity of anion exchanger having cyclic phenol sulfide sulfonic acid iron complex (IX) in oxidation with hydrogen peroxide:

Determination was repeatedly made three times by the same method as in Example 7, except that the cyclic phenol sulfide sulfonic acid iron complex (IX) was repeatedly used.

As a result, the absorbances observed in all the three measurements were almost the same. The same activity was reproduced even when the cyclic phenol sulfide sulfonic acid iron complex (IX) was repeatedly used. Consequently, the cyclic phenol sulfide sulfonic acid iron complex (IX) can withstand repetitions of use as a catalyst for oxidation by hydrogen peroxide.

### Example 9

### Production of ion exchanger having cyclic phenol sulfide sulfonic acid cerium complex (X) by preparing ion exchanger having cyclic phenol sulfide sulfonic acid beforehand and then incorporating cerium ion thereinto:

An aqueous solution prepared by dissolving 45.2 mg of the sodium cyclic phenol sulfide sulfonate (II) obtained in Production Example 2 in 50 ml of water was gradually added dropwise, under stirring, to a solution prepared by suspending 500 mg of an anion exchanger (DEAE cellulofine A-500, manufactured by Seikagaku Corporation) in 20 ml of water, followed by further stirring for 1 hour. The ion exchanger obtained was filtered off with a membrane filter, sufficiently washed with water, and then dried *in vacuo* over P₂O₅. The color of this ion exchanger having a cyclic phenol sulfide sulfonic acid supported thereon was white.

Subsequently, 10 ml of an aqueous solution prepared by dissolving Ce(NH₄)₂(NO₃)₆ in a concentration of 1.0×10⁻² M (considerably light yellow) was gradually added dropwise, under stirring, to a solution prepared by suspending 500 mg of the ion exchanger described above in 20 ml of water, followed by further stirring for 1 hour. The thus-obtained anion exchanger having a cyclic phenol sulfide sulfonic acid cerium complex (X) was filtered off with a membrane filter, sufficiently washed with water, and then dried *in vacuo* over P₂O₅. This anion exchanger having a cyclic phenol sulfide sulfonic acid cerium complex (X) supported thereon had a chocolate color.

The molar ratio of the cyclic phenol sulfide sulfonic acid to cerium ions in the thus-obtained ion exchanger having the cyclic phenol sulfide sulfonic acid cerium complex (X) was calculated from the Ce⁴⁺ ion concentration in 20 ml of the filtrate determined by conducting chelatometric titration with 0.01 M EDTA (ethylenediaminetetraacetic acid ester) solution, and was found to be 1:1.

### Example 10

### Production of complex of cyclic phenol sulfide sulfonic acid with cerium ion and of ion exchanger having the same:

To an aqueous solution prepared by dissolving 45.2 mg of the sodium cyclic phenol sulfide sulfonate (II) in 50 ml of water, 10 ml of an aqueous solution prepared by dissolving Ce(NH₄)₂(NO₃)₆ in a concentration of 1.0×10⁻² M was gradually added dropwise under stirring, followed by further stirring for 1 hour. The unreacted cerium ions were removed by adding 200 mg of a cation-exchange resin (Dowex MSC-1, manufactured by Muromachi Kagaku Kogyo Kaisha, Ltd.) to the reaction solution, further stirring the mixture for 1 hour, and filtering off the cation-exchange resin.

The complex (X) obtained is a cyclic phenol sulfide sulfonic acid cerium complex represented by formula (1) wherein X = H, Y = SO₃Na, Z = Sm, m = 1, n = 4, p = 1, q = 1, and M = Ce.

Subsequently, 50 ml of an aqueous solution of the cyclic phenol sulfide sulfonic acid cerium complex (X) described above was gradually added dropwise to a solution prepared by suspending 500 mg of an anion exchanger (DEAE cellulofine A-500, manufactured by Seikagaku Corporation) in 20 ml of water, followed by further stirring for 1 hour.

The ion exchanger obtained was filtered off with a membrane filter, sufficiently washed with water, and then vacuum-dried on P₂O₅. The amount of the cerium complex (X) supported was 1×10⁻⁴ mol per g of the anion exchanger.

### Example 11

### Deposition of cyclic phenol sulfide sulfonic acid cerium complex (X) on chitosan:

In 175 ml of 0.05 N hydrochloric acid solution was dissolved 0.5 g of chitosan (Chitosan 500, manufactured by Seikagaku Corporation). Thereafter, 280 ml of water was added to obtain an aqueous chitosan solution. A solution prepared by dissolving 45.2 mg of the sodium cyclic phenol sulfide sulfonic acid (II) obtained in Production Example 2 in 50 ml of water was gradually added dropwise to the chitosan solution with stirring at room temperature, followed by further stirring for 1 hour. An aqueous sodium bicarbonate solution was added to the resultant solution so as to make it neutral. The precipitate yielded was filtered off with a membrane filter, sufficiently washed with water, and then dried *in vacuo* over P₂O₅. The color of this chitosan having a cyclic phenol sulfide sulfonic acid supported thereon was white. Subsequently, 10 ml of an aqueous solution prepared by dissolving Ce(NH₄)₂(NO₃)₆ in a concentration of 1.0×10⁻² M was gradually added dropwise, under stirring at room temperature, to 20 ml of a solution prepared by suspending 0.5 g of the chitosan having a cyclic phenol sulfide sulfonic acid supported thereon in water, followed by further stirring for 1 hour. The thus-obtained chitosan having a cyclic phenol sulfide sulfonic acid cerium complex (X) was filtered off with a membrane filter, sufficiently washed with water, and then dried in *vacuo* over P₂O₅. This chitosan having a cyclic phenol sulfide sulfonic acid cerium complex (X) supported thereon had a chocolate color. Incidentally, the amount of the cerium complex (X) supported was 5.0×10⁻⁵ mol per 0.5 g of the chitosan.

### Example 12

### Catalytic activity of anion exchanger obtained in Example 10 having cyclic phenol sulfide sulfonic acid cerium complex (X) in hydrolysis of phosphodiester:

Sodium bis(p-nitrophenyl) phosphate (BNPP) was used as a phosphodiester. The presence of a p-nitrophenolate (λmax: 400 nm) as a hydrolyzate thereof in a reaction liquid was determined by determining the absorbance at 400 nm.

To a mixed solution consisting of 3 ml of a 2×10⁻⁴ M solution of BNPP and 3 ml of a buffer solution, 20 ml of the ion exchanger having a cyclic phenol sulfide sulfonic acid cerium complex (X) was added, followed by shaking at room temperature for 1 hour. The pH range was from 4 to 11. The buffer solution was prepared using 0.1 M CH₃COOH and 0.1 M CH₃COONa for pH ranges of from 4 to 6, prepared using 1/30 M KH₂PO₄ and 1/30 M Na₂HPO₄ for a pH of 7, or prepared using 0.1 M NH₄OH and 0.1 M NH₄Cl for pH ranges of from 8 to 11. Subsequently, the ion exchanger having a cyclic phenol sulfide sulfonic acid cerium complex (X) was filtered out of the reaction liquid with a membrane filter. The solution obtained was examined for absorbance at 400 nm.

The results are shown in Fig. 3.

As a result, absorption at 400 nm was observed, which indicated that the BNPP had been hydrolyzed in the presence of the anion exchanger having a cyclic phenol sulfide sulfonic acid cerium complex (X) and a p-nitrophenolate had been yielded. The absorption was maximum at around pH = 9.

### Example 13

### Catalytic activity of anion exchanger obtained in Example 9 having cyclic phenol sulfide sulfonic acid cerium complex (X) in hydrolysis of phosphodiester:

Absorbance at 400 nm was determined in the same manner as in Example 12, except that the anion exchanger obtained in Example 9 having a cyclic phenol sulfide sulfonic acid cerium complex (X) was used in place of the anion exchanger obtained in Example 10 having a cyclic phenol sulfide sulfonic acid cerium complex (X).

As a result, even when the anion exchanger obtained in Example 9 having a cyclic phenol sulfide sulfonic acid cerium complex (X) was used, the same absorbance was obtained as in the case where the anion exchanger obtained in Example 10 having a cyclic phenol sulfide sulfonic acid cerium complex (X) was used.

It can be seen that the same ion exchanger having a cyclic phenol sulfide metal complex was prepared in the case where the cyclic phenol sulfide metal complex was formed beforehand and the ion exchanger was prepared thereafter and in the case where an ion exchanger having a cyclic phenol sulfide was prepared first and the metal was incorporated thereafter.

### Example 14

### Catalytic activity of chitosan obtained in Example 11 having cyclic phenol sulfide sulfonic acid cerium complex (X) supported thereon in hydrolysis of phosphodiester:

Absorbance at 400 nm was determined in the same manner as in Example 12, except that the chitosan obtained in Example 11 having a cyclic phenol sulfide sulfonic acid cerium complex (X) supported thereon was used in place of the anion exchanger used in Example 12 having a cyclic phenol sulfide sulfonic acid cerium complex (X).

As a result, even when the chitosan having a cyclic phenol sulfide sulfonic acid cerium complex (X) supported thereon was used, the same results were obtained as the absorbance obtained in the case where the anion exchanger having a cyclic phenol sulfide sulfonic acid cerium complex (X) was used in Example 12.

### Example 15

### Reproducibility of catalytic activity of anion exchanger having cyclic phenol sulfide sulfonic acid cerium complex (X) in hydrolysis of phosphodiester:

An anion exchanger having a cyclic phenol sulfide sulfonic acid cerium complex (X) was prepared in the same manner as in Example 9, except that the metal was incorporated under conditions of 60°C in place of room temperature.

This ion exchanger having a cyclic phenol sulfide sulfonic acid cerium complex (X) was repeatedly used to repeatedly make determination five times at a pH of 9 by the same method as in Example 12.

The results are shown in Fig. 4.

As a result, the absorbances observed in all the five measurements were almost the same. The same activity was reproduced even when the ion exchanger having a cyclic phenol sulfide sulfonic acid cerium complex (X) was repeatedly used. Consequently, the anion exchanger having a cyclic phenol sulfide sulfonic acid cerium complex (X) can withstand repetitions of use as a catalyst for hydrolysis of phosphodiester.

### Industrial Applicability

The cyclic phenol sulfide metal complex of the present invention can be used in applications, such as catalysts for various chemical reactions, information/electronic materials, and the like. Furthermore, it has a catalytic activity in oxidations with hydrogen peroxide and an activity in the hydrolysis of phosphodiester. The method for analyzing hydrogen peroxide of the present invention can be used to conduct quantitative analysis for hydrogen peroxide.

## Claims

1. A method of oxidation by hydrogen peroxide, comprising reacting hydrogen peroxide in the presence of a cyclic phenol sulfide metal complex represented by formula (3): 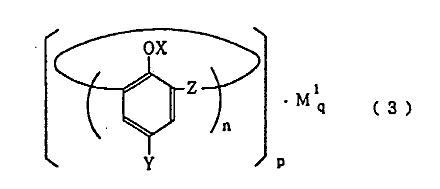 wherein X represents a hydrogen atom, a hydrocarbon group, a carboxyalkyl group, or a carbamoylalkyl group;
Y represents a hydrogen atom, a hydrocarbon group, a halogenated hydrocarbon group, a halogen atom, an acyl group, a hydroxyl group, a carboxyl group, an amido group, an amino group, a nitro group, a cyano group, a chlorosulfonic acid group, an alkoxysulfonyloxy group, or a sulfonic acid group;
Z represents Sm, SO, or SO₂;
m represents an integer of from 1 to 7;
n represents an integer of from 4 to 8;
M¹ represents a transition metal or a rear earth metal; and
p and q represent a composition ratio and an integer of 1 or more.

2. The method according to claim 1, wherein the cyclic phenol sulfide metal complex is mixed with or supported on a solid carrier.

3. A method of analysis for hydrogen peroxide, comprising detecting hydrogen peroxide by reacting the hydrogen peroxide in the presence of the cyclic phenol sulfide metal complex represented by formula (3) described in claim 1.

4. The method according to claim 3, wherein the cyclic phenol sulfide metal complex is mixed with or supported on a solid carrier.

5. Use of the cyclic phenol sulfide metal complex represented by formula (3) described in claim 1 as a catalyst for oxidation by hydrogen peroxide.
